# EUROPÄISCHE PATENTSCHRIFT

(11) **EP 2 066 796 B1**
(45) Veröffentlichungstag und Bekanntmachung des Hinweises auf die Patenterteilung: **29.06.2011**
(21) Anmeldenummer: 07723377.3
(22) Anmeldetag: 19.03.2007
(51) Int. Cl.: C12P 5/02, C07C 7/11, B01D 53/14

(54) **VERFAHREN UND VORRICHTUNG ZUR TRENNUNG VON METHAN UND KOHLENDIOXID AUS BIOGAS**
METHOD AND DEVICE FOR SEPARATING METHANE AND CARBON DIOXIDE FROM BIOGAS
ProcÉdÉ et dispositif de séparation du méthane et du dioxyde dE carbone dU biogaz

(30) Priorität: 20.09.2006 DE 102006044192
(43) Veröffentlichungstag der Anmeldung: 10.06.2009
(73) Patentinhaber: MT-Biomethan GmbH, 27404 Zeven (DE)
(72) Erfinder: GÜNTHER, Lothar, 82538 Geretsried (DE)
(74) Vertreter: Tragsdorf, Bodo
(86) Internationale Anmeldenummer: PCT/EP2007/002410
(87) Internationale Veröffentlichungsnummer: WO 2008/034473

(56) Entgegenhaltungen:
- DE-B3-102005 051 952
- DE-U1- 20 300 663
- US-A- 4 409 102
- BISHNUPADA MANDAL ET. AL.: "Simultaneous Absorption of CO2 and H2S Into Aqueous Blends of N-Methyldiethanolamine and Diethanolamine" ENVIRON. SCI. TECHNOL., Bd. 40, Nr. 19, 19. August 2006 (2006-08-19), Seiten 6076-6084, XP002444694

## Beschreibung

Die Erfindung betrifft ein Verfahren zur Trennung von Methan und Kohlendioxid aus Biogas und eine Vorrichtung zur Durchführung des Verfahrens. Diese sind zur Reinigung von Blogas bestimmt, wobei aus dem Biogas Kohlendioxid abgeschieden wird.

Die bekannten Biogase haben folgende Zusammensetzungen:

| | |
|---|---|
| Methan | 40 bis 70 Vol.-% |
| Wasserstoff | bis 2 Vol.-% |
| CO₂, | bis 60 Vol.-% |
| Stickstoff | bis 5 Vol.-% |
| Sauerstoff | bis 2 Vol.-% |
| H₂O | 2 bis 4 Vol.-% |
| H₂S | 0,01 bis 0,6 Vol.-% |

Nach den bekannten Verfahren zur Abtrennung von Kohlendioxid und Reinigung von Raffinerlegas wird dieses über Druckwäsche bei einem Druck von 10 bis 30 bar mittels unterschiedlichen Waschlösungen, wie Aminen, H₂O, Methanol oder Aceton, bei Temperaturen von 10 bis 40 °C in einer Absorptionskolonne behandelt, wobei die im Raffineriegas enthaltenen Anteile von Kohlendioxid in einer Waschlösung gebunden werden. Die verwendete, aus der Absorptionskolonne austretende und mit Kohlendioxid beladene Waschlösung wird anschließend auf einen Druck von 1 bis 5 bar entspannt und einer Desorptlonskolonne zugeführt, in der unter Zuführung von Wärme die Austreibung des in der Waschlösung enthaltenen Kohlendioxids erfolgt. Das abgeschiedene Kohlendioxid wird aus der Desorptionskoionne in die Umgebung abgeleitet oder durch nachfolgende Reinigung und Kompression weiter verflüssigt. Dafür sind ebenfalls Verfahren bekannt. Das mit den verwendeten Verfahren gereinigte Gas enthält überwiegend Methan und Wasserstoff, wobei Kohlendioxid bis auf unter 20 ppm abgetrennt werden kann.

Je nach verwendetem Waschmittel ist es auch möglich, neben dem Kohlendioxid gleichzeitig H₂O, COS (organische Schwefelverbindungen) oder SO₂ abzuschelden. Diese Komponenten sind dann Im abgeschiedenen Kohlendioxid enthalten.

Weiterhin ist bekannt, dass nach dem gleichen Prinzip die Reinigung eines Prozessgases bei der Herstellung von Ammoniak mittels Pottasche als Waschmittel erfolgt. Hier wird das abgetrennte Kohlendioxid gasförmig bei einem Druck bis zu 5 bar in die Umgebung abgeleitet.

Zur Trennung von Methan und CO₂ aus Biogas ist bekannt (DE 203 00 663 U1), Biogas in einem Flüssigkeftsringkompressor mit einer Waschflüssigkeit (Polyethylenglykolether) zu vermischen, das Gas/Flüssigkeitsgemisch auf einen Druck von 8 bar zu komprimieren und das verdichtete Gas/Flüssigkeitagemiech einer Adsorptionskoionne mit einer Schüttung aus Edelstahlkörpem zuzuführen, in der Polyethylenglykolether als Sperrflüssigkeit versprüht wird. Dabei werden Kohlendioxid und Schwefelwasserstoff in der Waschflüssigkeit physikalisch gebunden. Die Waschflüssigkeit sammelt sich am Kolonnensumpf an und das komprimierte Methangas wird am Kopf der Kolonne abgezogen. Die abgetrennte Waschflüssigkeit wird entspannt, regenerativ aufbereitet und wieder in die Adsorptionakolonne zurückgeführt.

Bei einer physikalischen Wäsche unter Druck treten Druckverluste auf und hohe Blomethankonzentratlonen führen zu Methangasverlusten. Außerdem wird keine vollständige Abtrennung von CO₂ erreicht. Bei der Auslegung und Herstellung der Absorptionskolonne sind spezielle sicherhettstechnische Anforderungen zu berücksichtigen, die zu einem höheren Aufwand und zusätzlichen Kosten führen.

Soll das nach den bekannten Verfahren unter Druck gereinigte Biogas, das Biomethan, in ein Erdgasnetz eingespeist werden, so muss dieses wieder entspannt werden, da im Erdgasnetz ein wesentlich geringerer Druck vorliegt. Diese Verfahrensweise ist energetisch unwirtschaftlich.

Bekannt sind auch Untersuchungen zu kinetischen Modellen zur Absorption von CO₂ und H₂S aus sauren Gasen in einer Kolonne mittels einer aminhaltigen Waschlösung, wobei als saures Gas auch Biogas genannt ist (Bishnupada, Environ. Sci. Technol. 2006, 40, 6076 bis 6084). Die Untersuchungen zur Absorption wurden unter Normaldruck und Normaltemperatur in einer Fallfilmkolonne durchgeführt.

Der Erfindung liegt die Aufgabe zugrunde, ein Verfahren zur Trennung von Methan und CO₂ aus Biogas zu schaffen, das sich durch eine energetisch günstige Betriebsweise auszeichnet. Ferner soll eine zur Durchführung des Verfahrens geeignete Vorrichtung geschaffen werden.

Erfindungsgemäß wird die Aufgabe durch die im Anspruch 1 angegebenen Merkmale gelöst. Vorteilhafte Weiterbildungen der Verfahrensweise sind Gegenstand der Ansprüche 2 bis 10. Eine zur Durchführung des Verfahrens geeignete Vorrichtung ist in Anspruch 11 angegeben. Die Ansprüche 12 bis 14 beziehen sich auf weitere Ausgestaltungen der Vorrichtung.

Das gegebenenfalls vorgereinigte Biogas wird einer Absorptionskoionne zugeführt, in der bei Normaldruck und Normaltemperatur das im Biogas enthaltene Kohlendioxid entfernt wird. Das Biogas ist am Austritt der Absorptionskolonne frei von Kohlendioxid bzw. es wurde ein bestimmter Restgehalt an CO₂ eingestellt. Für eine Absorption unter Normaldruck und Normaltemperatur ist es von Vorteil, wenn die Füllkörper eine möglichst große Oberfläche aufweisen. Vorzugsweise besitzt die Füllkörperschüttung eine Oberfläche von 600 bis 1200 m²/m³. Die Raumbelastung beträgt 5 bis 40 Nm³/m³h. Während des Aufsteigens des Biogases durch die Füllkörperschüttung wird Im Biogas enthaltenes Kohlendioxid durch Chemosorption in der Waschflüssigkeit gebunden. Die Kontaktzeit zwischen der Waschflüssigkeit und dem Biogas sollte mindestens 40 Sekunden, vorzugsweise 50 bis 400 Sekunden, betragen, um eine hohe Reinheit des Methangases zu erzielen.

Die jeweilige Kontaktzeit ist abhängig von der Kohlendioxidkonzentration im Biogas und der Aminkonzentration in der Waschlösung. Die Temperatur des Biogases und der Waschlösung haben hier im Wesentlichen im Bereich von 10 bis 50 °C keinen Einfluss.

Die Einstellung der Kontaktzeit erfolgt durch eine geregelte Biomethanabsaugung aus dem Wäscher über einen Verdichter, in Abhängigkeit von dem beabsichtigten Reingaswert für CO₂, der gemessen wird.

Biogas lässt sich nach der vorgeschlagenen Verfahrensweise besonders wirtschaftlich in Methan und CO₂ trennen. Es treten keine Methanverluste auf und CO₂ kann bis auf geringe Mengen im ppm-Bereich vollständig abgetrennt werden. Der energetische Aufwand für eine gegebenenfalls notwendige Verdichtung des Methangases ist Im Vergleich zu einer Druckwäsche um ca. 50% geringer, da das CO₂ bereits nahezu vollständig abgetrennt ist und die zu verdichtende Gasmenge somit deutlich geringer ist.

Die Strömungsgeschwindigkeit des Biogases wird beim Eintritt in die Absorptionskolonne bezogen auf den freien Kolonnenquerschnitt auf 0,01 bis 0,1 m/s eingestellt.

Als Waschflüssigkeit wird vorzugsweise eine Aminlösung eingesetzt, die beispielsweise aus in Wasser gelöstem Diethanolamin mit einer Konzentration von 20 Gew.-% besteht. Die eingesetzte Waschlösung besitzt in etwa die gleiche Temperatur wie das zugeführte Biogas. Dies hat den Vorteil, dass der Wasserhaushalt in der Waschkolonne konstant gehalten werden kann. Dadurch wird außerdem sichergestellt, dass sich die Aminkonzentration nicht ändert.

Nach der Absorption in der Waschflüssigkeit noch gebundenes Kohlendioxid wird anschließend unter höherem Druck von 2 bis 30 bar und einer Temperatur von mindestens 120 °C mittels Desorption entfernt. Zur Desorption kann als Wärmetauschermedium Dampf oder Thermalöl verwendet werden.

Um Methangas mit einer höheren Reinheit, z.B. von über 99,5% zu erhalten, ist es zweckmäßig, das Biogas vor der Zuführung in die Absorptionskolonne mittels geeigneter Wäsche und/oder Adsorption zu reinigen, um in diesem enthaltene Anteile an NH₃, COS (organische Schwefelverbindungen), H₂S und SO₂ zu entfernen.

Das aus der Absorptionskolonne drucklos abgeführte Biomethan kann über eine Trocknung sowie Polizeifilter geleitet werden und anschließend entweder in ein Erdgasnetz eingespeist oder einer anderen stofflichen Verwertung zugeführt werden.

Für eine, gegebenenfalls erforderliche, Verdichtung des Biomethans können Verdichter aus Normalstahl verwendet werden.

Eine zur Durchführung des Verfahrens geeignete Vorrichtung sollte so ausgelegt sein, dass die erforderlichen Einbauten in der Absorptionskolonne, die Füllkörperschüttung, eine Oberfläche von 600 bis 1.200 m²/m³ besitzt und einen Raumbelastungsfaktor von 5 bis 40 Nm³/m³h, vorzugsweise 20 Nm³/m³h. Die einzelnen Füllkörper sollten einen mittleren Durchmesser von 5 bis 8 mm haben. Eine Raumbelastung bzw. ein Raumbelastungsfaktor von 5 bis 40 Nm³/m³ h bedeutet, dass für eine der Absorptionskolonne zugeführte Menge von 5 bis 40 Nm²/h Blogas mindestens 1 m³ Reaktionsvolumen notwendig ist. Das Reaktionsvolumen wird durch die eingesetzten Füllkörper bestimmt, die vergleichsweise eine extrem große Oberfläche besitzen. Derart kleine Füllkörper, die eine große Kontaktoberfläche ergeben, sind für den Einsatz im Rahmen einer Druckwäsche vollkommen ungeeignet. Ausgehend von der erforderlichen großen Oberfläche der Fülikörperschüttung (600 bis 1200 m²/m³) lassen sich über eine geregelte Methangasabsaugung die Absaug- bzw. Strömungsgeschwindigkeit des Methans und die Verweil- bzw. Kontaktzeiten des Biogases im Wäscher, z.B. von 50 bis 400 Sekunden, einstellen, um eine nahezu vollständige Entfernung des CO₂ zu gewährleisten. Erforderlichenfalls kann auch ein gewünschter Restgehalt an CO₂ im Methangas eingestellt werden.

in Abhängigkeit von der zu reinigenden Blogasmenge und dem erforderlichen Reaktionsvolumen der Füllkörperschüttung sollte die als zylindrischer Behälter ausgeführte Absorptlonskolonne ein Länge/Durchmesser-Verhältnis von 4 bis 20 auf, vorzugsweise 10 bis 20 bzw.14 bis 18 aufweisen. Von Vorteil ist auch, dass im Vergleich zu einer Druckwäsche die Kolonnenhöhe verringert werden kann. Das UD-Verhältnis der Absorptionskolonne ist im Wesentlichen von der zu behandelnden Biogasmenge abhängig.

Die vorgeschlagene Absorptionskolonne zur Entfernung von CO₂ entspricht in ihrem Grundaufbau einem Wäscher. Aufgrund der speziellen Ausführung der Füllkörperschüttung entspricht die Prozessführung des Biogases Innerhalb der Kolonne, zur Abtrennung von CO₂ durch Chemosorption, der Funktionsweise eines Reaktors.

Die aus der Absorptionskolonne mit einer Temperatur von 10 bis 50 °C austretende Waschlösung wird auf einen Druck von 2 bis 30 bar komprimiert, mittels konventioneller Regeneration durch Wärmezuführung auf eine Temperatur von 120 bis 180 °C, vorzugsweise bis 160 °C, erwärmt und einer Desorptionskolonne/Flashentspannung zugeführt. Hier erfolgt die Abtrennung von Kohlendioxid und Wasserdampf. Die gereinigte Waschlösung wird wieder zur drucklosen Waschkolonne zurückgeleitet.

Mit der drucklosen Biomethanerzeugung, bei der ca. 50 % an Verdichterleistung eingespart werden, können kostengünstige Werkstoffe verwendet werden. Dies ist bei einer Druckwäsche oder einem anderen Druckreinigungsverfahren nicht möglich.

Die Erfindung wird nachstehend an zwei Beispielen erläutert.

### Beispiel 1:

Zur Trennung von Methan und CO₂ aus Biogas wird eine Absorptions- bzw. Waschkolonne eingesetzt, die eine Länge von 12 m und einen Durchmesser von 1,75 m besitzt. Als Einbauten ist eine Füllkörperschüttung angeordnet, die aus Füllkörpern mit einem mittleren Durchmesser von 5 bis 8 mm und einer spezifischen Oberfläche von 800 m²/m³ besteht. Die Füllkörper bestehen aus Kunststoff, vorzugsweise Polypropylen.

Die oberhalb des Sumpfes angeordnete Füllkörperschüttung weist eine Kolonnenschütthöhe von 8 m auf. Das Kolonnenvolumen der Füllkörperschüttung beträgt somit 19,23 m³. Ausgehend von der Auslegung der Kolonne mit einem LD-Verhältnis von 4,57 stellt sich bei einer Zuführung von 500 Nm³/h (N=Normzustand) Biogas am Kolonneneintritt eine Strömungsgeschwindigkeit des zu reinigenden Biogases, bezogen auf den freien Kolonnenquerschnitt, von 0,062 m/s ein.

Unter vorgenannten Bedingungen ergibt sich ein Raumbelastungsfaktor von 26,3. Unterhalb des Bodens der Kolonne werden in diese 500 Nm³/h Biogas mit einer Temperatur von 25 °C und einem Druck von 1015 mbar eingetragen. Das zugeführte, vorgereinigte Biogas besitzt folgende Zusammensetzung:

| | |
|---|---|
| CH₄ | 53,5 Vol.-% |
| CO₂ | 44,0 Vol.-% |
| H₂O | 2,5 Vol.-% |
| H₂S | 150 ppm |

Am Kopf der Waschkolonne wird als Waschmedium eine aminhaltige Waschlösung, bestehend aus Wasser und Diethanolamin, mit einer Konzentration von 20 Gew.-%, mit einer Temperatur von 25 °C eingesprüht, in einer Menge von 15 m³/h.

Die Waschlösung, wird im Kreislauf gefahren und nach erfolgter Regenerierung, Insbesondere der Abtrennung von Resten an CO₂ und H₂S, wieder eingesetzt.

Im Gegenstromverfahren durchströmt das drucklos zugeführte Biogas <die Füllkörperschüttung und gelangt dabei mit der Waschlösung in Kontakt. Die Kontaktzeit zur nahezu vollständigen Abtrennung von im Biogas enthaltenem CO₂ und Schwefelverbindungen beträgt 138 s. Dabei werden CO₂ und Schwefelverbindungen in der Waschlösung chemisch gebunden.

Das am Kopf der Waschkolonne gebildete Methangas wird mittels eines nachgeschalteten Verdichters abgezogen. Gleichzeitig wird im Methangas die CO₂-Konzentration gemessen und in Abhängigkeit von dem gemessenen Wert die Drehzahl des Verdichters geregelt. Ober die Drehzahl des Verdichters wird die Kontaktzeit innerhalb der Waschkolonne eingestellt. Das am Kolonnenaustritt abgetrennte Methan wird mit einer Strömungsgeschwindigkeit von 0,033 m/s abgezogen und hat folgende Zusammensetzung:

| | |
|---|---|
| CH₄ | 97,3 Vol.-% |
| CO₂ | 0,2 Vol.-% |
| H₂O | 2,5 Vol.-% |
| H₂S | 1 ppm |

### Beispiel 2

Zur Trennung von Methan und CO₂ aus Biogas wird eine Absorptions- bzw. Waschkolonne eingesetzt, die eine Länge von 12 m und einen Durchmesser von 0,45 m besitzt. Als Einbauten ist eine Fülikörperschüttung angeordnet, die aus Füllkörpern mit einem mittleren Durchmesser von 5 bis 8 mm und einer spezifischen Oberfläche von 800 m²/m³ besteht. Die Füllköper bestehen aus Kunststoff, vorzugsweise aus Polyethylen.

Die oberhalb des Sumpfes angeordnete Füllkörpemchüttung weist eine Kolonnenschütthöhe von 8 m auf. Das Kolonnenvolumen der Füllkörperschüttung beträgt somit 1,27 m². Ausgehend von der Auslegung der Kolonne mit einem L/D-Verhältnis von 17,8 stellt sich bei einer Zuführung von 25 Nm²/h (N=Normzustand) Biogas am Kolonneneintritt eine Strömungsgeschwindigkeit des zu reinigenden Biogases, bezogen auf den freien Kolonnenquerschnitt, von 0,043 m/s ein.

Unter vorgenannten Bedingungen ergibt sich ein Raumbelastungsfaktor von 19,7. Unterhalb des Bodens der Kolonne werden in diese 25Nm³/h Biogas mit einer Temperatur von 28°C und einem Druck von 1015 mbar eingetragen. Das zugeführte Biogas besitzt folgende Zusammensetzung:

| | |
|---|---|
| CH₄ | 51,1 Vol.-% |
| CO₂ | 46,0 Vol.-% |
| H₂O | 2,9-Vol.-% |
| H₂S | 80 ppm |

Am Kopf der Waschkolonne wird als Waschmedium eine aminhaltige Waschlösung, bestehend aus Wasser und Diethanolamin mit einer Konzentration von 30 Gew.-%, mit einer Temperatur von 15°C eingesprüht, in einer Menge von 0,45 m³/h.

Die Waschlösung wird im Kreislauf gefahren und nach erfolgter Regenerierung, Insbesondere der Abtrennung von Resten an CO₂ und H₂S, wieder eingesetzt.

Im Gegenstromverfahren durchströmt das drucklos zugeführte Biogas die Fülikörperschüttung und gelangt dabei mit der Waschlösung in Kontakt. Dabei werden im Biogas enthaltenes CO₂ und Schwefelverbindungen nahezu vollständig abgetrennt und in der Waschlösung gebunden. Das am Kopf der Waschkolonne gebildete Methangas wird mittels eines nachgeschalteten Verdichters abgezogen. Gleichzeitig wird im Methangas die CO₂-Konzentration gemessen und in Abhängigkeit von dem gemessenen Wert die Drehzahl des Verdichters geregelt. Ober die Drehzahl des Verdichters wird die Kontaktzeit innerhalb der Waschkolonne eingestellt. Im vorliegenden Beispiel wird zur vollständigen Entfernung von CO₂ über den Verdichter eine Kontaktzeit von 184 s eingestellt. Das am Kolonnenaustritt abgetrennte Methan wird mit einer Strömungsgeschwindigkeit von 0,025 m/s abgezogen und hat folgende Zusammensetzung:

| | |
|---|---|
| CH₄ | 98,4 Vol.-% |
| CO₂ | 0,1 Vol.-% |
| H₂O | 1,5 Vol.-% |
| H₂S | 0,1 ppm |

Durch eine Entfeuchtung des Methans auf einen Restgehalt von 50 mg/Nm³ an Wasser wird ein hochreines Methan mit folgender Zusammensetzung erhalten:

| | |
|---|---|
| CH₄ | 99,80 Vol.-% |
| CO₂ | 0,12 Vol.-% |
| H₂O | 50 mg/Nm³ |
| H₂S | 0,1 ppm |

Im entfeuchteten Methan sind nur noch von 0,08 Vol.-% Wasserstoff und CO₂ enthalten. Das Methangas kann somit ohne weitere Behandlung in ein örtliches Erdgas-Versorgungsnetz eingespeist oder einer anderweitigen Verwendung zugeführt werden.

## Patentansprüche

1. Verfahren zur Trennung von Methan und Kohlendioxid aus Biogas mittels Wäsche in einer Absorptionskolonne in der Biogas im Gegenstrom zu einer zugeführten, Kohlendioxid und Schwefelwasserstoff bindenden Aminlösung als Waschflüssigkeit unter Normaldruck und Normaltemperatur aufsteigt, wobei das Biogas eine Absorptionskolonne mit Fülikörperschüttung und einer Raumbelastung von 5 bis 40 Nm²/m³h durchströmt und dabei im Biogas enthaltenes Kohlendioxid durch Chemosorption in der Waschflüsalgkeit gebunden wird, und das Methangas am Kopf der Absorptionakolonne mit einer definierten Strömungsgeschwindigkeit abgezogen wird, und zur nachfolgenden Regenerierung der Waschflüsalgkeit in dieser noch gebundenes Kohlendioxid unter höherem Druck von 2 bis 30 bar und einer Temperatur von mindestens 120 °C mittels Desorption entfernt wird.

2. Verfahren nach Anspruch 1, **dadurch gekennzeichnet, dass** das Biogas in der Absorptionskolonne eine Füllkörperschüftung mit einer Oberfläche von 600 bis 1200 m²/m³ und einer Raumbelastung von 5 bis 40 Nm³/m³h durchströmt.

3. Verfahren nach einem der Ansprüche 1 oder 2, **dadurch gekennzeichnet, dass** für die Wäsche eine Kontaktzeit von mindestens 40 Sekunden, vorzugsweise 50 bis 400 Sekunden, eingestellt wird.

4. Verfahren nach einem der Ansprüche 1 bis 3, **dadurch gekennzeichnet, dass** die CO₂-Konzentration im Methangas gemessen wird und in Abhängigkeit von dem ermittelten Wert die Strömungsgeschwindigkeit des abgezogenen Methangases und damit die Kontaktzeit eingestellt wird.

5. Verfahren nach einem der Ansprüche 1 bis 4, **dadurch gekennzeichnet, dass** die Strömungsgeschwindigkeit des Biogases, bezogen auf den freien Kolonnenquerschnitt, auf 0,01 bis 0,1 m/s eingestellt wird.

6. Verfahren nach einem der Ansprüche 1 bis 5, **dadurch gekennzeichnet, dass** über die Aminkonzentration der Waschflüssigkeit die Kontaktzeit beeinflusst wird.

7. Verfahren nach einem der Ansprüche 1 bis 6, **dadurch gekennzeichnet, dass** über die Einstellung der Biogastemperatur und der Temperatur der im Kreislauf gefahrenen Waschflüssigkeit der Wassergehalt der Waschflüssigkeit konstant gehalten wird.

8. Verfahren nach einem der Ansprüche 1 bis 7, **dadurch gekennzeichnet, dass** das Biogas und die zugeführte Waschlösung gleiche Temperaturen aufweisen.

9. Verfahren nach einem der Ansprüche 1 bis 8, **dadurch gekennzeichnet, dass** die Desorption unter einem Druck von 8 bis 20 bar und bei Temperaturen von bis zu 180 °C erfolgt.

10. Verfahren nach einem der Ansprüche 1 bis 9, **dadurch gekennzeichnet, dass** im Biogas enthaltene Anteile an NH₃, COS, H₂S und SO₂ bereits vor der Zuführung des Biogases in die Absorptionskolonne entfernt werden.

11. Vorrichtung zur Durchführung des Verfahrens nach mindestens einem der vorhergehenden Ansprüche bestehend aus mindestens einer Absorptionskolonne mit einer Fülikörperschüttung, **dadurch gekennzeichnet, dass** die Füllkörperschüttung eine Oberfläche von 600 bis 1200 m²/m³ und eine Raumbelastung von 5 bis 40 Nm³/m³h aufweist.

12. Vorrichtung nach Anspruch 11, **dadurch gekennzeichnet, dass** das Länge/Durchmesserverhältnis des Behälters 4 bis 20, vorzugsweise 10 bis 20, beträgt.

13. Vorrichtung nach einem der Ansprüche 11 oder 12, **dadurch gekennzeichnet, dass** die Füllkörper der Füllkörperschüttung einen mittleren Durchmesser von 5 bis 8 mm aufweisen.

14. Vorrichtung nach einem der Ansprüche 11 bis 13, **dadurch gekennzeichnet, dass** der Absorptionskolonne ein Verdichter zur Einstellung der Absauggeschwindigkeit des Methans und der Kontaktzeit des Biogases nachgeschaltet ist.

## Claims

1. Method of separating methane and carbon dioxide from biogas by scrubbing in an absorption column where the biogas ascends under atmospheric pressure and standard temperature in counter flow to an amine solution as washing liquid, which binds carbon dioxide and hydrogen sulphide, in which the biogas in the absorption column flows through a packed bed with a volumetric loading of 5 up to 40 Nm³/m³h and wherein the carbon dioxide present in the biogas is bound in the washing liquid by chemosorption and the methane gas is separated off at the top of the absorption column at a defined flow velocity, and for subsequent regeneration of the washing liquid the carbon dioxide still bound in these is subsequently removed by desorption at a relatively high pressure of 2 to 30 bar and a temperature of at least 120°C.

2. Method in accordance with claim 1 **characterised in that** the biogas in the absorption column flows through a packed bed with a surface area of 600 to 1200 m²/m³ and a volumetric loading of 5 up to 40 Nm³/m³h.

3. Method in accordance with one of the claims 1 or 2 **characterised in that** a contact time of at least 40 seconds, preferably 50 to 400 seconds, is set for scrubbing.

4. Method in accordance with one of the claims 1 to 3 **characterised in that** the CO₂ concentration in the methane gas is measured and the flow velocity of the removed methane gas, and thus the contact time, is set according to the value determined.

5. Method in accordance with one of the claims 1 to 4 **characterised in that** the flow velocity of the biogas based on the available column cross-section is set at 0.01 to 0.1 m/s.

6. Method in accordance with one of the claims 1 to 5 **characterised in that** the contact time is affected by the amine concentration of the wash liquid.

7. Method in accordance with one of the claims 1 to 6 **characterised in that** the water balance of the wash liquid is kept constant by setting the biogas temperature and the temperature of the wash liquid circulated in the circuit.

8. Method in accordance with one of the claims 1 to 7 **characterised in that** the biogas and the washing solution fed in are at the same temperature.

9. Method in accordance with one of the claims 1 to 8 **characterised in that** desorption takes place at a pressure of 8 to 20 bar.

10. Method in accordance with one of the claims 1 to 9 **characterised in that** the proportions of NH₃, COS, H₂S and SO₂ present in the biogas are removed before the biogas is fed into the absorption column.

11. Device for carrying out the process in accordance with at least one of the previous claims consisting of at least an absorption column with a packed bed **characterised in that** the packed bed has a surface area of 600 to 100 m²/m³ and a space velocity of 5 to 40 N³/m³h.

12. Device in accordance with claim 11 **characterised in that** the length/diameter ratio of the vessel is 4:20, preferably 10:20.

13. Device in accordance with one of the claims 11 or 12 **characterised in that** the packing of the packed bed has an average diameter of 5 to 8 mm.

14. Device in accordance with one of the claims 11 to 13 **characterised in that** a compressor for setting the extraction speed of the methane and the contact time is positioned downstream of the absorption column.

## Revendications

1. Procédé pour la séparation du méthane et du dioxyde de carbone contenus dans le biogaz au moyen d'un lavage dans une colonne d'absorption dans laquelle le biogaz s'élève dans le courant inverse, sous forme de liquide lavant, à pression normale et à température normale vers une solution d'amine rajoutée et liant le dioxyde de carbone et l'hydrogène sulfuré, le biogaz parcourant une colonne d'absorption à garnissage d'une charge volumique comprise entre 5 et 40 Nm³/m³h, le dioxyde de carbone contenu dans le biogaz étant lié dans le liquide lavant par chimisorption, et le méthane étant évacué à la tête de la colonne d'absorption à une vitesse de courant définie, et le dioxyde de carbone encore lié dans le liquide lavant étant éliminé par désorption à une pression plus élevée comprise entre 2 et 30 bar et à une température d'au moins 120°C en vue de la régénération consécutive dudit liquide lavant.

2. Procédé selon la revendication 1, **caractérisé en ce que** le biogaz parcourt dans la colonne d'absorption un garnissage d'une surface comprise entre 600 et 1 200 m²/m³ et d'une charge volumique comprise entre 5 et 40 Nm³/m³h.

3. Procédé selon l'une quelconque des revendications 1 ou 2, **caractérisé en ce que**, pour le lavage, un temps de contact d'au moins 40 secondes, de préférence entre 50 et 400 secondes, est réglé.

4. Procédé selon l'une quelconque des revendications 1 à 3, **caractérisé en ce que** la concentration en CO₂ dans le méthane est mesurée et la vitesse de courant du méthane évacué et ainsi, le temps de contact, sont réglés en fonction de la valeur déterminée.

5. Procédé selon l'une quelconque des revendications 1 à 4, **caractérisé en ce que** la vitesse de courant du biogaz est réglée entre 0,01 et 0,1 m/s par rapport à la section transversale libre de la colonne.

6. Procédé selon l'une quelconque des revendications 1 à 5, **caractérisé en ce que** le temps de contact est influencé par l'intermédiaire de la concentration en amine du liquide lavant.

7. Procédé selon l'une quelconque des revendications 1 à 6, **caractérisé en ce que** la teneur en eau du liquide lavant est maintenue à un niveau constant en réglant la température du biogaz et la température du liquide lavant en circulation.

8. Procédé selon l'une quelconque des revendications 1 à 7, **caractérisé en ce que** le biogaz et la solution lavante rajoutée présentent la même température.

9. Procédé selon l'une quelconque des revendications 1 à 8, **caractérisé en ce que** la désorption est effectuée à une pression comprise entre 8 et 20 bar et à des températures allant jusqu'à 180°C.

10. Procédé selon l'une quelconque des revendications 1 à 9, **caractérisé en ce que** les parts de NH₃, COS, H₂S et SO₂ contenues dans le biogaz sont éliminées avant le rajout du biogaz dans la colonne d'absorption.

11. Dispositif pour la mise en oeuvre du procédé selon au moins l'une quelconque des revendications précédentes, se composant au moins d'une colonne d'absorption à garnissage, **caractérisé en ce que** le garnissage présente une surface comprise entre 600 et 1 200 m²/m³ et une charge volumique comprise entre 5 et 40 Nm³/m³h.

12. Dispositif selon la revendication 11, **caractérisé en ce que** le rapport longueur/diamètre du récipient est compris entre 4 et 20, de préférence entre 10 et 20.

13. Dispositif selon l'une quelconque des revendications 11 ou 12, **caractérisé en ce que** les corps de remplissage du garnissage présentent un diamètre moyen compris entre 5 et 8 mm.

14. Dispositif selon l'une quelconque des revendications 11 à 13, **caractérisé en ce qu'**un compresseur pour le réglage de la vitesse d'aspiration du méthane et du temps de contact du biogaz est placé à la suite de la colonne d'absorption.
